# EUROPEAN PATENT SPECIFICATION

(11) **EP 0 495 869 B1**
(45) Date of publication and mention of the grant of the patent: **03.01.1996**
(21) Application number: 90915513.7
(22) Date of filing: 15.10.1990
(51) Int. Cl.: A61F 2/04, A61L 27/00

(54) **POLYMER PRODUCTS**
POLYMERPRODUKTE
PRODUITS POLYMERES

(30) Priority: 18.10.1989 GB 8923516
(43) Date of publication of application: 29.07.1992
(73) Proprietor: PolyMedica Industries, Inc., Woburn, MA 01801 (US)
(72) Inventor: CHARLESWORTH, David Ollerton Lodge School Lane, Cheshire WA16 8SD (GB); UNDERWOOD, Christopher, John 22 Keswick Avenue, Manchester M34 3QD (GB); CHIAN, Kerm, Sin 22 Fulbrook Road Bevington, Wirral L63 9HT (GB)
(74) Representative: McNeight, David Leslie
(86) International application number: GB9001591
(87) International publication number: WO9105522

(56) References cited:
- EP-A- 0 269 254
- US-A- 4 605 406
- US-A- 4 731 073
- US-A- 4 798 607

## Description

This invention relates to methods for making polymer products and to novel products made according to the methods.

Polymer products in the form of vascular prostheses conventionally comprise a conduit having varying dimensions and mechanical characteristics which are as close as materials and manufacturing processes will allow to the vessel in the body whose function it is intended that the prosthesis should replace.

US-A-4 605 406 discloses one method of fabricating prosthesis material having a conduit configuration and in particular discloses a method by which polymers are precipitated on the interior walls of tubes or other cylindrical conduits.

A number of tubular prostheses may be grafted into a single vascular system.

There is merit, at least conceptually, in attempting to maintain as much as possible of the host vascular tissue during surgical procedures which involve vascular replacement, principally because there is less alien material introduced into the patient.

Polymer products which comprise a branch and arms emanating therefrom may typically be produced by Joining the arms to an independently fabricted branch region. Whilst this procedure has the advantage that it allows the construction of relatively complex branched structures, disadvantages include the product lacking a relatively uniform mechanical consistency; together with a relatively time consuming and thus expensive preparative procedure.

Where a vascular system branches, tubular prostheses may be grafted onto each of the arms comprised thereby. This technique suffers from the serious disadvantage in that it necessitates joins at the increased number of junctions between host and prosthetic vascular material. Consequently, the time spent by the patient under anaesthetic and subject to cardiopulmonary bypass is increased which increases the likelihood of the development of pulmonary and circulatory system disorders, together with the raised possiblilty of cardiac ischaemia, necrosis and infarct.

Moreover, there is a finite possibility that a prosthesis will fail mechanically at the region of its attachment to host vascular material, conventionally regarded as the weakest and most sensitive region of the graft. An increased number of such attachment regions in a single vascular system synergistically increases the possibility of failure of the prosthetic vascular system as a whole.

The present invention provides inter alia methods of producing vascular products, particularly in the form of prostheses, which overcome the disadvantages and deficiencies which characterise prior art vascular prosthetic products.

According to the present invention there is provided a method of forming a polymer product comprising a luminate vessel and a sheet attached to said luminate vessel at one end thereof so that the luminate vessel is open at that end, characterised by precipitating on to said luminate vessel a sheet of polymer from a solution comprising an organic solvent and precipitable polymer and forming an aperture in said sheet, said aperture communicating with the lumen of the vessel.

The invention also includes products made according to the aforementioned methods.

The polymer may comprise between 17 per cent and 30 per cent by weight of the solution comprising said polymer and solvent.

The product may exhibit approximately a 20 per cent shrinkage during the manufacture thereof..

The product may be formed from an existing luminate vessel, itself formed from a solution chemically similar or identical to said solution.

The polymer may be biocompatible and may comprise a vascular prosthesis.

The prosthesis may comprise a graft adapted for use in a part of a vascular system comprising branches therein, such as, for example, that part of the aorta from which the coronary arteries arise.

Said solution may further comprise a porosifier which may be insoluble in said solution but soluble in aqueous systems.

The porosifier may comprise a carbonate, such as, for example, sodium hydrogen carbonate.

Said porosifier may have an average particle size of 50 to 100 microns, and may comprise between 10 and 60 per cent by weight of the solution.

Said solution may further comprise a surfactant.

The surfactant may be an anionic detergent, such as, for example, an alkoxy sulphite.

Said surfactant may be an alkaline metal salt of dodecyl sulphate, such as sodium dodecyl sulphate.

Said surfactant may comprise between 0.1 and 10 per cent by weight of said solution.

The wall thickness of said product may be 0.5 to 1.5 millimetres, and if said product comprises a luminate vessel, the lumenal diameter thereof may be 3 to 30 mm.

The polymer of which said product is comprised may be polyurethane.

Said polyurethane may be a linear segmented poly(ether)urethane with a number average molecular weight in the region of 20 to 100 kDa.

The invention will be further apparent from the following description and several figures of the accompanying drawings, which illustrate, by way of example only, methods of forming polymer products, according to the invention and polymer products made according to the methods.

Of the figures :-
- Figure 1: shows a method of producing a polymer vascular prosthesis from a plurality of luminate vessels;
- Figure 2: shows a second method of forming a polymer vascular prosthesis according to the invention, in which said prosthesis is precipitated onto the surface of a multi-tubular former;
- and Figure 3: shows a polymer product in the form of a vascular prosthesis in situ comprising a region of the aorta from which the coronary arteries arise, together with a region of each of said arteries.

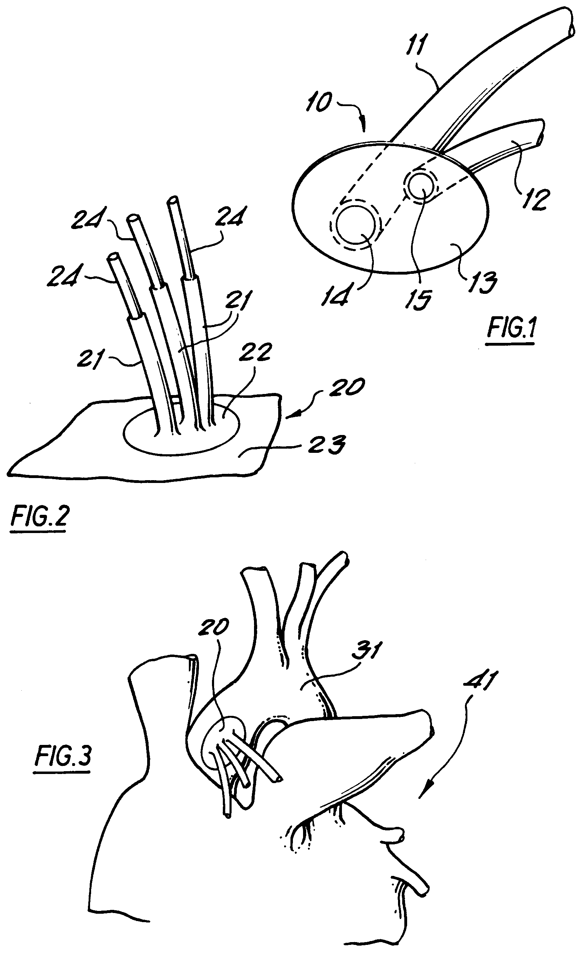

Figures 1 and 2 illustrate methods of forming polymer products in the form of prosthetic grafts adapted for use in a region of a vascular system comprising branches therein.

As shown in Figure 1, the prosthesis 10 is formed from two luminate vessels 11,12 by precipitating thereonto a sheet 13 of polymer from a solution comprising an organic solvent and precipitable polymer. Apertures 14,15 are formed in the sheet 13, so that there is a fluid communication between the sheet 13 and the lumen of each vessel.

In a second method of forming a vascular prosthesis, the prosthesis 20 comprises a plurality of luminate vessels 21 having a sheet 22 of polymer therearound and is formed by precipitating onto a product former 23 comprising a plurality of tubular conduits 24 a layer of polymer from a solution comprising an organic solvent and precipitable polymer

Figure 2 shows the prosthesis 20 of Figure 2 as a graft in the aorta 31 of a human heart, shown partially at 41.

The polymer can comprise at least 17 per cent but less than 30 per cent by weight of the solution comprising said polymer and solvent and the polymer can exhibit approximately a 20 per cent shrinkage during the precipitation thereof.

Preferably, where the product is formed from an existing luminate vessel, as shown in Figure 1, the solution from which the vessel is formed is chemically similar or identical to the solution comprising organic solvent and precipitable polymer from which solution said sheet is precipitated.

Vascular protheses necessarily should be made from biocompatible material, and the polymer of which said product is comprised is a polyurethane, characterised by being a linear segmented poly(ether)urethane with a number average molecular weight in the region of 20 to 100 kDa.

It is desirable that prostheses for use in the blood vascular system should have pores in their walls, preferably relatively large on the external surface, and relatively small on the luminal surface of the prosthesis.

Such pores enable formation of pseudointima by endothelial cells particularly, but also pericytes and other cells normally found in the vascular architecture. Such cells can present a non-thrombogenic surface to blood flowing through the prosthesis and, additionally, release factors which are ordinarily non-thrombogenic, and platelet anti-aggregators and anti-thrombogenic derivatives of arachidonic acid.

Preferably, the solution from which the prosthesis is precipitated further comprises a porosifier, such as sodium hydrogen carbonate, which is insoluble therein but soluble, for example, in an aqueous system.

Said porosifier has an average particle size of 50 to 100 microns, and comprises between 10 and 60 per cent by weight of the solution.

A surfactant is added to the solution to modulate further the porosity of the walls, particularly at the precipitation surfaces.

Although the surfactant can comprise between 0.1 and 10 per cent by weight of said solution the preferred concentration is about 2 per cent.

The wall thickness of the prosthesis corresponds to the thicknesses of the vessels found in the body and which it is intended that the prosthesis should replace.

Alternatively, a wall thickness of the prosthesis can be determined from an analysis of the physio-mechanical requirements that must be met by the prosthesis, with relatively little regard to the wall thickness thereof.

Typically, the wall thickness of the prosthesis is 0.5 to 1.5 millimetres, and the lumenal diameter thereof is 3 to 30 mm.

Although the present invention has been described in conjunction with particular embodiments, it will be appreciated by those skilled in the art that various other changes, omissions and additions thereto may be made without departing from its scope as described herein.

For example, the polymer products may comprise bio-compatible sheets having pores, the sheets acting as matrices into which cells may migrate in tissue culture. Such sheets may be of use in skin grafts, for example.

The polymer products may be used as filters and selectively permeable membranes.

## Claims

1. A method of forming a polymer product (10, 20) comprising a luminate vessel (11, 12, 21) and a sheet (13, 22) attached to said luminate vessel at one end thereof so that the luminate vessel is open at that end, characterised by precipitating on to said luminate vessel a sheet of polymer (13, 22) from a solution comprising an organic solvent and precipitable polymer and forming an aperture (14, 15) in said sheet, said aperture communicating with the lumen of the vessel

2. A method according to claim 1, in which the polymer comprises at least 17 per cent but less than 30 per cent by weight of the solution comprising said polymer solvent.

3. A method according to claim 1 and 2, in which during formation of said product there is approximately a 20 per cent shrinkage.

4. A method according to any preceding claim, in which the polymer is bio-compatible.

5. A method according to any one of claims 1 to 4, in which the wall thickness of said sheet is 0.5 to 1.5 millimetres

6. A method according to any one of claims 1 to 5, in which the diameter of the lumen of said vessel is 3 to 30 mm.

7. A method according to any one claims 1 to 6, in which the polymer comprises polyurethane.

8. A method according to any one of the preceding claims, in which the product comprises a vascular prosthesis.

9. A method according to claim 8, in which the prosthesis comprises a graft adapted for use in a part of a vascular system comprising branches therein.

10. A method according to claim 9, in which the prosthesis comprises a graft adapted to replace that part of the aorta from which the coronary arteries exit.

## Patentansprüche

1. Verfahren zur Bildung eines Polymerproduktes (10,20), welches ein hohles Gefäß (11,12,21) und ein an einem Ende des hohlen Gefäßes derart befestigtes Blatt (13,22) aufweist, daß das hohle Gefäß an diesem Ende offen ist, gekennzeichnet durch Niederschlagen eines Blattes aus Polymer (13,22) auf das hohle Gefäß aus einer Lösung, die ein organisches Lösungsmittel und ein abscheidbares Polymer aufweist, und durch Bilden einer Öffnung (14,15) in dem Blatt, wobei die Öffnung mit dem Hohlraum des Gefäßes verbunden ist.

2. Verfahren nach Anspruch 1, bei welchem das Polymer zumindest 17 Gewichtsprozent, aber weniger als 30 Gewichtsprozent der das Polymer-Lösungsmittel aufweisenden Lösung aufweist.

3. Verfahren nach Anspruch 1 und 2, bei welchem während der Bildung des Produktes angenähert eine 20-prozentige Schrumpfung auftritt.

4. Verfahren nach jedem der vorhergehenden Ansprüche, bei welchem das Polymer biokompatibel ist.

5. Verfahren nach einem der Ansprüche 1 bis 4, bei welchem die Wanddicke des Blattes 0,5 bis 1,5 Millimeter beträgt.

6. Verfahren nach einem der Ansprüche 1 bis 5, bei welchem der Durchmesser des Hohlraums des Gefäßes 3 bis 30 mm beträgt.

7. Verfahren nach einem der Ansprüche 1 bis 6, bei welchem das Polymer Polyurethan aufweist.

8. Verfahren nach einem der vorhergehenden Ansprüche, bei welchem das Produkt eine Gefäßprothese aufweist.

9. Verfahren nach Anspruch 8, bei welchem die Prothese ein Transplantat aufweist, das für die Verwendung in einem Teil eines Gefäßsystems mit Abzweigungen darin angepaßt ist.

10. Verfahren nach Anspruch 9, bei welchem die Prothese ein Transplantat aufweist, das für den Ersatz des Teils der Aorta, von dem die Kranzarterien austreten, angepaßt ist.

## Revendications

1. Procédé de formation d'un produit polymère (10, 20) comprenant un vaisseau à lumière (11, 12, 21) et une feuille (13, 22) fixée audit vaisseau à lumière à une de ses extrémités, de sorte que le vaisseau à lumière est ouvert à cette extrémité, caractérisé en ce qu'on précipite sur ledit vaisseau à lumière une feuille de polymère (13, 22) à partir d'une solution comprenant un solvant organique et un polymère précipitable et qu'on forme une ouverture (14, 15) dans ladite feuille, ladite ouverture communiquant avec la lumière du vaisseau.

2. Procédé selon la revendication 1, dans lequel le polymère comprend au moins 17 % mais moins de 30 % en poids de la solution comprenant ledit polymère et ledit solvant.

3. Procédé selon la revendication 1 et 2, dans lequel on observe un retrait d'approximativement 20 % durant la formation dudit produit.

4. Procédé selon l'une quelconque des revendications précédentes, dans lequel le produit est biocompatible.

5. Procédé selon l'une quelconque des revendications 1 à 4, dans lequel l'épaisseur de paroi de ladite feuille est comprise entre 0,5 et 1,5 mm.

6. Procédé selon l'une quelconque des revendications 1 à 5, dans lequel le diamètre de la lumière dudit vaisseau est compris entre 3 et 30 mm.

7. Procédé selon l'une quelconque des revendications 1 à 6, dans lequel le polymère comprend du polyuréthane.

8. Procédé selon l'une quelconque des revendications précédentes, dans lequel le produit comprend une prothèse vasculaire.

9. Procédé selon la revendication 8, dans lequel la prothèse comprend un greffon conçu pour être utilisé dans une partie d'un système vasculaire comprenant des ramifications.

10. Procédé selon la revendication 9, dans lequel la prothèse comprend un greffon conçu pour remplacer la partie de l'aorte d'où partent les artères coronaires.
